# EUROPEAN PATENT APPLICATION

(11) **EP 4 393 473 A1**
(43) Date of publication of application: **03.07.2024**
(21) Application number: 23170529.4
(22) Date of filing: 28.04.2023
(51) Int. Cl.: A61K 9/00, A61K 31/416, A61K 31/58, A61K 9/19, A61K 9/16

(54) **MANUFACTURING METHOD OF MEDICATION**

(30) Priority: 30.12.2022 TW 111150775
(71) Applicant: Yung Shin Pharmaceutical Ind. Co., Ltd., Tachia, Taichung 43744 (TW)
(72) Inventor: Lee, Fang-Yu, 437 Taichung City (TW); Yu, Yu-Shyang, 407 Taichung City (TW)
(74) Representative: Straus, Alexander

(57) **Abstract**

A manufacturing method of medication includes the following steps. Firstly, an effective dosage of an API is dissolved in a dissolved agent to form a first solution, wherein the API includes triamcinolone acetonide or YC-1 compound. Next, the first solution is filtered through a filter membrane at a room temperature to obtain a second solution. Then, a precipitating agent is added into the second solution to obtain a third solution, followed by continuously stirring the third solution to precipitate crystals. The third solution is further stirred under the room temperature, a low pressure to sequentially remove the dissolved agent and the precipitating agent. Finally, a volume of the crystals is quantified to primary package and to refill the crystals, to obtain the medication.

## Description

### Field of the Invention

The present disclosure relates to a manufacturing method of a medication, and in particular, to a manufacturing method of an injection.

### Background of the Invention

Common drug dosage forms may be divided into drops, ointments, injections, gel and the like, in which, the injections are generally used by directly delivering itself to the blood system till reaching an effective dosage, so as to obtain the advantages like quick and effective response. However, the product qualities of the injections, as well as the manufacture thereof, are both critical for the medication industry. Firstly, the injections requested to be highly stable and lower toxicity, and also, the weight and the particle size of the prepared powder or freeze-dried powder are required to be uniform and microminiaturized at the same time, thereby dramatically reducing any possible irritation of the drug. In addition, the raw materials of the injections, as well as the manufacturing processes (including preparation, secondary packaging, transportation, primary packaging, and filling) for processing the row materials have to be free from contamination, to sufficiently avoid any residue such as the impurity being less than or equal to 100 micrometers remained therein to damage to the product quality. Therefore, there is an urgent need of overcoming all the limits and the disadvantages of the manufacturing method of the injection, in order to meet the practical requirements in the pharmaceutical industry.

### Summary of the Invention

This in mind, the present disclosure aims at providing a manufacturing method of a medication, in which an active pharmaceutical ingredient (API) is processed by using bi-solvent for crystal precipitation, so as to effectively avoid the possible contaminations and to dramatically control the particle size of the medication. In this way, the medication obtained thereby in the present disclosure enables to meet the various product requirements like less contamination, lower toxicity, and finer particle, thereby achieving better product quality.

This achieved by a manufacturing method of a medication according to claims. The dependent claims pertain to corresponding further developments and improvements.

As will be seen more clearly from the detailed description following below, a manufacturing method of a medication is provided by the present disclosure. The manufacturing method of a medication includes the following steps. Firstly, an effective dosage of an active pharmaceutical ingredient is dissolved in a dissolved agent to form a first solution, wherein the active pharmaceutical ingredient includes triamcinolone acetonide or a YC-1 compound. Next, the first solution is filtered through a filter membrane, at a room temperature to obtain a second solution. Then, a precipitating agent is added into the second solution to obtain a third solution, followed by continuously stirring the third solution to precipitate crystals. The third solution is further stirred under the room temperature and a lower pressure to sequentially remove the dissolved agent and the precipitating agent from the third solution. Finally, a volume of the crystals is quantified to primary package and to refill, and a lyophilization process is performed on the crystals to obtain the medication, wherein the triamcinolone acetonide is represented by structure of formula (I): 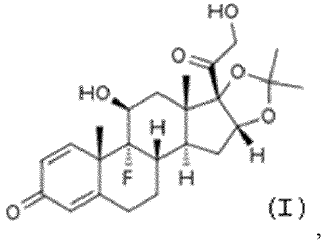 and the YC-1 compound is represented by structure of formula (II):

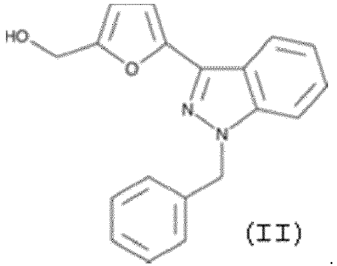

Overall speaking, according to the manufacturing method of a medication of the present disclosure, a special crystal precipitation technique and a lyophilization process are combined in use to precisely define the particle size of precipitated crystals, in which, the multi-solvents are applied to the active pharmaceutical ingredients for crystal precipitation, and the lyophilization process is carried out when the crystal is in a semi-solid dosage form, thereby simplifying the manufacturing process of the medication, and effectively preventing from any external contamination at the same time. Thus, the medication obtained from the manufacturing method of a medication of the present disclosure enables to gain better product quality.

### Brief Description of the Drawings

FIG. 1 is a schematic diagram illustrating a particle size distribution (PSD) of a product obtained in a manufacturing method of a medication according to a first embodiment of the present disclosure.
FIG. 2 is a schematic diagram illustrating a particle size distribution of another product obtained in a manufacturing method of a medication according to the first embodiment of the present disclosure.
FIG. 3 to FIG. 8 are schematic diagrams illustrating a particle size distribution of a product obtained in a manufacturing method of a medication according to a second embodiment of the present disclosure, in which:

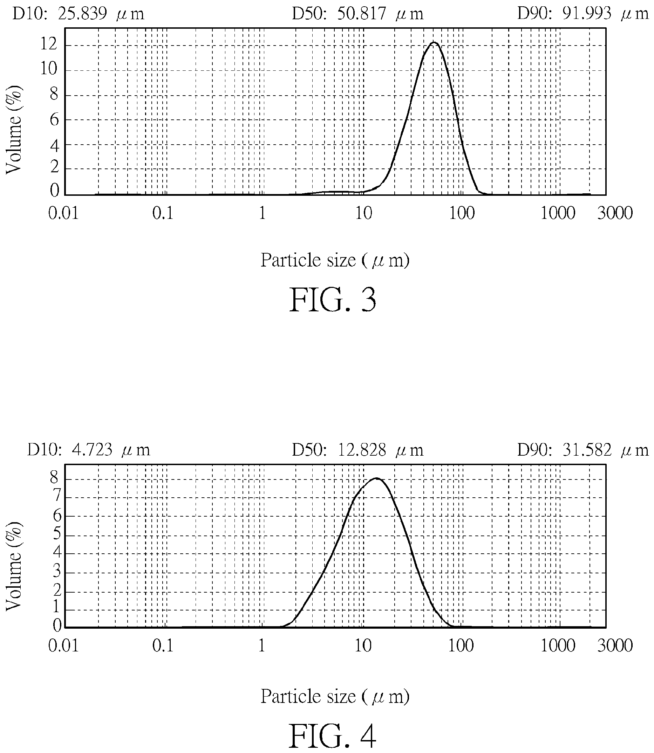

   FIG. 3 illustrates a particle size distribution of a product obtained in Example 1;
   FIG. 4 illustrates a particle size distribution of a product obtained in Example 2;

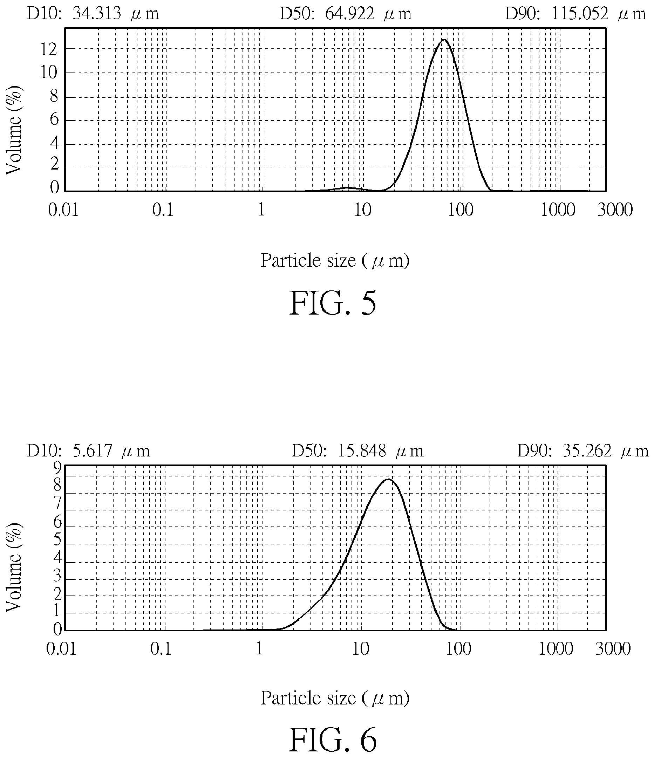

   FIG. 5 illustrates a particle size distribution of a product obtained in Example 3;
   FIG. 6 illustrates a particle size distribution of a product obtained in Example 4;

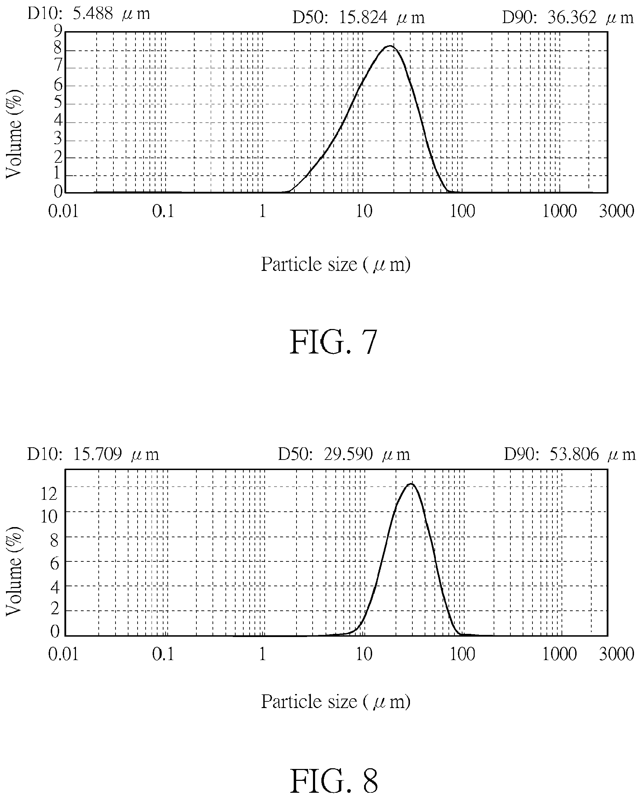

   FIG. 7 illustrates a particle size distribution of a product obtained in Example 5; and
   FIG. 8 illustrates a particle size distribution of a product obtained in Example 6.

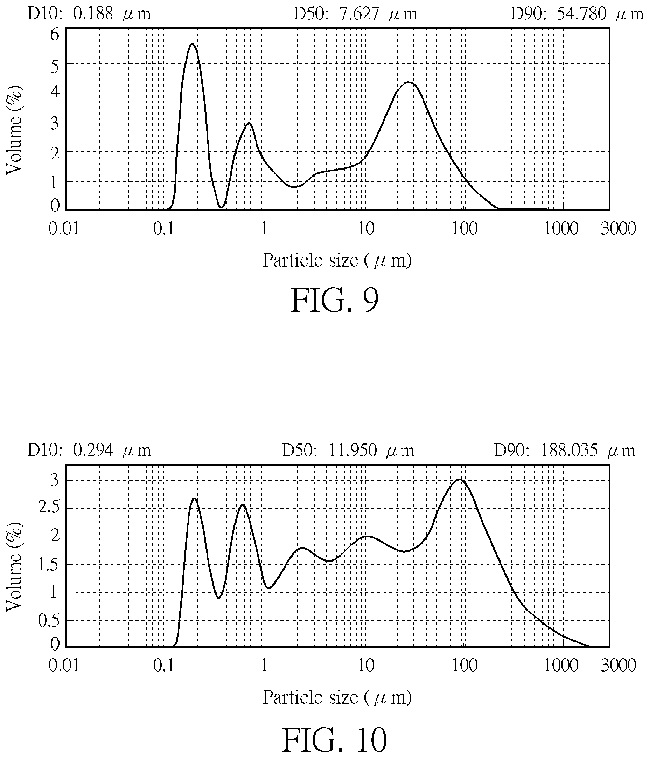

FIG. 9 to FIG. 14 are schematic diagrams illustrating a particle size distribution of another product obtained in a manufacturing method of a medication according to the second embodiment of the present disclosure, in which:
   FIG. 9 illustrates a particle size distribution of a product obtained in Example 7;
   FIG. 10 illustrates a particle size distribution of a product obtained in Example 8;

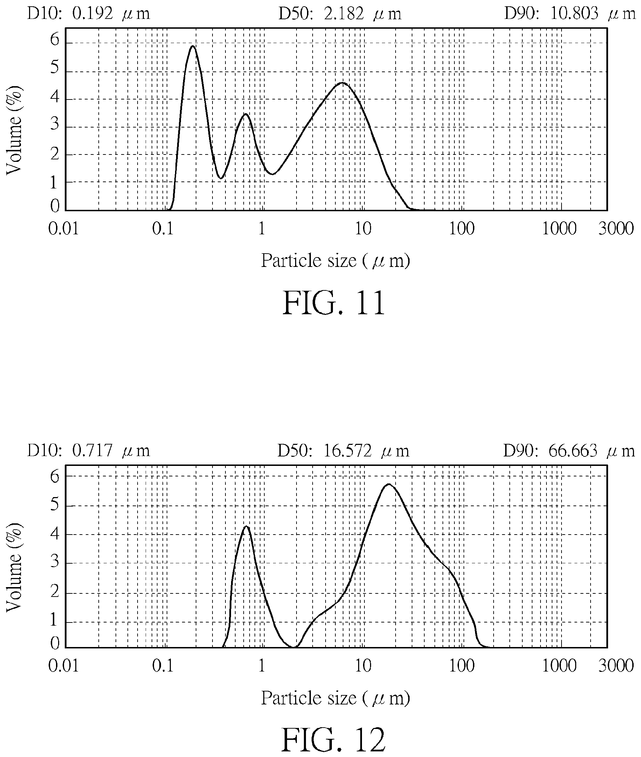

   FIG. 11 illustrates a particle size distribution of a product obtained in Example 9;
   FIG. 12 illustrates a particle size distribution of a product obtained in Example 10;

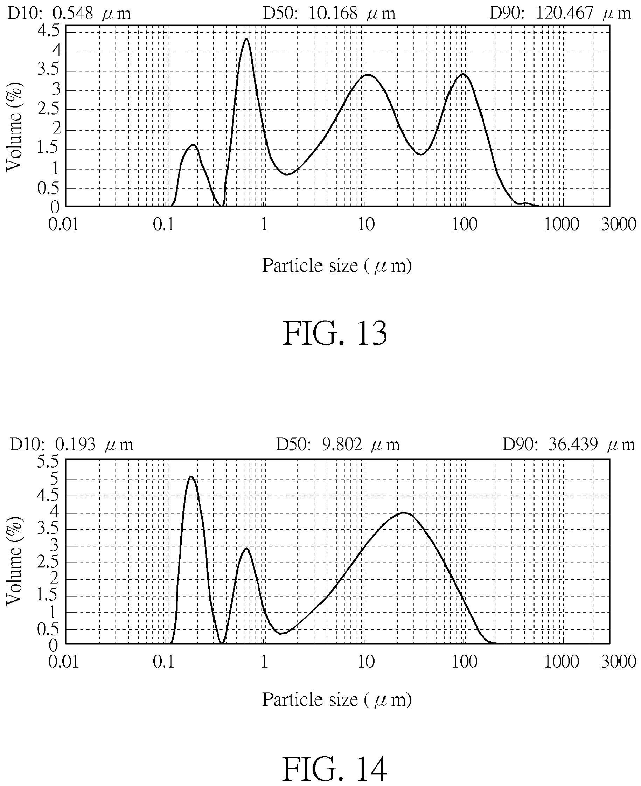

   FIG. 13 illustrates a particle size distribution of a product obtained in Example 11; and
   FIG. 14 illustrates a particle size distribution of a product obtained in Example 12.

### Detailed Description

To provide a better understanding of the presented disclosure, preferred embodiments will be described in detail. The preferred embodiments of the present disclosure are illustrated in the accompanying drawings with numbered elements. In addition, the technical features in different embodiments described in the following may be replaced, recombined, or mixed with one another to constitute another embodiment without departing from the spirit of the present disclosure.

In the present disclosure, the term "about" or "substantial" generally means within 20%, preferably within 10%, and more preferably within 5%, 3%, 2%, 1%, or 0.5% of a given value or range. Unless otherwise expressly specified, all of the numerical ranges, amounts, values and percentages disclosed herein should be understood as modified in all instances by the term "about" or "substantial". Accordingly, unless indicated to the contrary, the numerical parameters set forth in the present disclosure and attached claims are approximations that can vary as desired.

A manufacturing method of a medication according to a first embodiment includes but is not limited to be the following steps. Firstly, an active pharmaceutical ingredient (API) is provided, and an effective dosage of the API is processed through a dry powder filling technology, with the API being refilled by a sterile dry powder, followed by quantify a volume or a weight thereof for primary packaging. Then, the medication of the first embodiment is obtained thereby, wherein the API for example includes any pharmaceutical raw material which is used to treat eye diseases, such as triamcinolone acetonide, YC-1 compound (CAS#: 170632-4), an analogues or a composition of the aforementioned pharmaceutical raw materials, or other ingredients which is required to be manufactured into an injection. The triamcinolone acetonide is represented by the structure of formula (I): 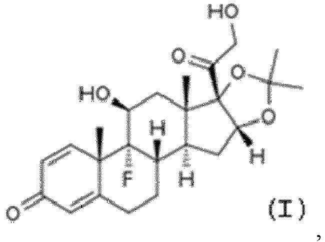 and the YC-1 compound is represented by the structure of formula (II) :

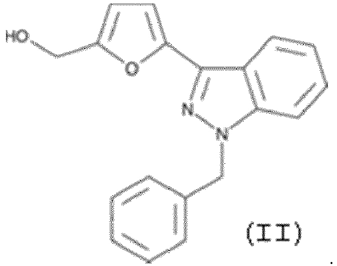

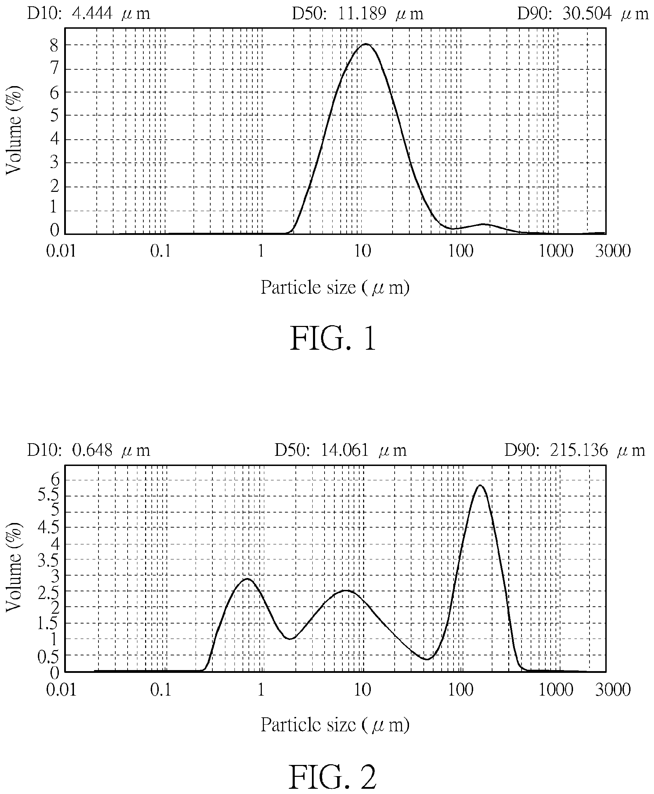

When the API includes triamcinolone acetonide, a particle size distribution (PSD) of crystals within the medication is about 1 micrometers (µm) to 100µm, with 10% of the crystals having a particle size (D10) of less than 4.444µm, with 50% of the crystals having a particle size (D50) of less than 11.189µm, and with 90% of the crystals having a particle size (D90) of less than 30.504, as shown in FIG. 1, to meet a good manufacturing practice (GMP) standard for medication. On the other hand, when the API includes the YC-1 compound, a particle size distribution (PSD) of crystals within the medication is about 0.5µm to 250µm, with 10% of the crystals having a particle size (D10) of less than 0.648µm, with 50% of the crystals having a particle size (D50) of less than 14.061µm, and with 90% of the crystals having a particle size (D90) of less than 215.136µm, as shown in FIG. 2, also to meet the GMP standard for medication.

Through these arrangements, the medication obtained from the manufacturing method of the first embodiment is allowable to be used as an injection, in particular being used as a powder suspension referring to a drug which is generally stored in a sterile container, and may be further dissolved in a proper liquid to form a suspension, to meet the GMP standard for medication. However, people well-skilled in the art should easily realize the manufacturing method of the medication in the present disclosure is not limited to be the aforementioned embodiment, and may further include other examples or variations under practical product requirements. The following description will detail the different embodiments of the manufacturing method of the medication in the present disclosure. To simplify the description, the following description will detail the dissimilarities among the different embodiments and the identical features will not be redundantly described. In order to compare the differences between the embodiments easily, the identical components in each of the following embodiments are marked with identical symbols.

According to a second embodiment of the present disclosure, another manufacturing method of a medication is provided, to precisely control the particle size of the medication, and to further improve the disadvantages caused by the aforementioned dry powder filling technology, such as easily leading to the contamination like hairs, impurities or other residues during sampling the APIs, or the weight variation during the filling process. Then, the manufacturing method of the second embodiment is allowable to obtain a medication with uniform and finer particles under a more sufficient and simplified process flow, also being free from any external contamination. According to the manufacturing method of the medication in the second embodiment of the present disclosure, a special crystal precipitation technique and a lyophilization process are combined in use to precisely define the particle size, and which includes but not limited to the following steps. Please refer to Table 1, a preparation step (S1) is firstly carried out by dissolving an effective amount of an API in a dissolved agent to form a first solution, wherein the API for example includes any pharmaceutical raw material which is used to treat eye diseases, such as triamcinolone acetonide, antibiotic drugs, a YC-1 compound (CAS#: 170632-4), an analogues or a composition of the aforementioned pharmaceutical raw materials, or other ingredients which is required to be manufactured into an injection. The triamcinolone acetonide is represented by the structure of formula (I): 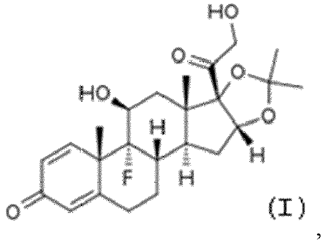 and the YC-1 compound is represented by the structure of formula (II)

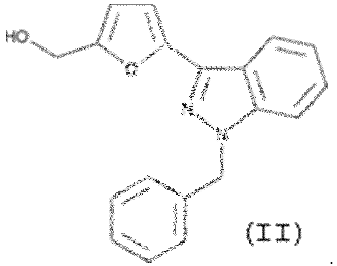

The dissolved agent may be any kind of the solvents according to the characteristics of the API, such as a suitable, lower toxic organic solvent. In the present embodiment, the dissolved agent for example includes a (C1-C4) alcohol or a (C1-C4) ketone, preferably being selected from a group consisting of methanol, ethanol, isopropanol, tertiary butanol (TBA) and acetone, but not limited thereto. In the present embodiment, each 10 milligrams (mg) of the API is for example dissolved in 0.5-0.8 milliliters (ml) of the dissolved agent, preferably being 0.65 ml, but not limited thereto. When the volume of the dissolved agent is too low, the API may not be dissolved completely, thereby resulting in poor yield, and however, when the volume of the dissolved agent is too high, the API may therefore obtain poor crystallization efficiency, as well as a poor uniformity of the particle size thereby.

Next, a sterilization step (S2) is performed. In the preset embodiment, a sterile filtration process is carried out by filtering the first solution through a filter membrane to obtain a second solution, which is a sterile filtrate. The aperture of the filter membrane is about 0.22-0.45µm, preferably being about 0.22µm, but not limited thereto. In addition, the sterilization step (S2) is preferably performed at a room temperature, for example, being about 20-30 degrees Celsius, and more preferably being about 25 degrees Celsius, but not limited thereto.

Also, a precipitation step (S3) is performed, in which a precipitating agent is added into the second solution to obtain a third solution, with a volume ratio of the precipitating agent to the second solution being about 2:1, followed by continuously stirring the third solution for a period of time, such as one hour, to precipitate crystals from the third solution. In the present embodiment, the precipitating agent for example includes pure water or other suitable liquids with low toxicity, but is not limited thereto. In particular, each 10 mg of the API is precipitated by about 0.5-1.5 ml of the precipitating agent, preferably being about 1 ml, but not limited thereto.

Then, a first concentration step (S4) and a second concentration step (S5) are sequentially performed, by continuously stirring the third solution at the room temperature (20-30 degrees Celsius) and a gradually reduced pressure, to remove the dissolved agent and the precipitating agent from the third solution respectively, thereby obtaining the crystals. According to the first concentration step (S4), the third solution is stirred under a lower pressure being about -720 to -685 mmHg for 20-24 hours, to remove the dissolved agent from the third solution, and also, according to the second concentration step (S5), the third solution is continuously stirred under another lower pressure being about -730 to -710 mmHg for 12 to 16 hours, to remove the precipitating agent from the third solution, wherein the pressure range of the second concentration step (S5) is preferably lower than that of the first concentration step (S4), so as to precisely control the particle size and uniformity of the crystals. Then, the medication obtained subsequently may therefore achieve uniform and microminiaturized particle size at the same time.

After that, a primary packaging step (S6) and a drying step (S7) are sequentially performed, in which the third solution is continuously stirred, followed by quantifying a volume of the crystals to primary packaging and refilling the crystals. The volume of the crystals is about 2-4 ml but is not limited thereto. Then, a lyophilization process and a vacuum concentration process are sequentially performed, to further remove the residual precipitating agent from the third solution, thereby obtaining the medication. In the present embodiment, the lyophilization process is carried out under a low pressure and a gradually increasing temperature, to remove the precipitating agent from the third solution, for example, being performed under a pressure of about 0.5 mmHg, and a temperature gradually increasing from -35 degrees Celsius to zero degrees Celsius and then to 35 degrees Celsius (-35°C→0°C→35°C) for about 22 to 24 hours, but not limited thereto. On the other hand, the vacuum concentration process is performed under the room temperature (20-30°C) and a pressure ranged from -720 to -685 mmHg for about 12 to 16 hours, to remove the residual precipitating agent from the third solution.

**Table 1: The manufacturing method of the medication according to the second embodiment**

| | processes | description |
|---|---|---|
| S1 | preparation step | dissolving an effective amount of an API in a dissolved agent to form a first solution |
| S2 | sterilization step | filtering the first solution through a filter membrane, under the room temperature (20-30°C), to obtain a second solution |
| S3 | precipitation step | adding a precipitating agent into the second solution to obtain a third solution, and continuously stirring the third solution for a period of time to precipitate crystals from the third solution |
| S4 | first concentration step | continuously stirring the third solution at the room temperature (20-30 degrees Celsius) and a low pressure (-720 to -685mmHg) to remove the dissolved agent |
| S5 | second concentration step | continuously stirring the third solution at the room temperature (20-30 degrees Celsius) and another low pressure (-730 to -710mmHg), to remove the precipitating agent |
| S6 | primary packaging step | continuously stirring the third solution and quantifying a volume of the crystals to primary packaging the crystals |
| S7 | drying step | sequentially performing the lyophilization process and the vacuum concentration process to remove the residual precipitating agent from the third solution |

Through these arrangements, the medication obtained from the manufacturing method of the second embodiment is also allowable to be used as an injection, in particular being used as a powder suspension referring to a drug which may be further dissolved in a proper liquid to form a suspension, to meet the GMP standard for medication. In this way, the manufacturing method of the present embodiment enable to process water indissoluble APIs such as triamcinolone acetonide or the YC-1 compound, to manufacture into injections which can be fast absorbed and utilized by human or animals, in particular the injections with higher purity, less contamination and lower toxicity. For example, when the API includes triamcinolone acetonide, the medication obtained thereby is allowable to be used as an injection for a treatment of vitreous visualization in ophthalmic vitrectomy, or diabetic macular edema, with a sufficient amount of saline or ocular perfusate being added into a bottle containing an effective dose of the medication followed by shaking the bottle sufficiently to form a suspension, and with the suspension being injected to the target portion.

Based on the manufacturing method of the medication of the present embodiment, an aseptic crystal precipitation technique and a lyophilization process are combined in use to precisely define the particle size of precipitated crystals, in which, the bi-solvents (including the dissolved agent and the precipitating agent) are both applied to the APIs for crystal precipitation. Precisely, the APIs are firstly dissolved in the dissolved agent (in the preparation step S 1), followed by sequentially performing the sterilization (in the sterilization step S2) and the precipitation (in the precipitation step S3) processes, to precipitate the crystals, and then, the multi-stepped vacuum concentration process (including the first concentration step S4 and the second concentration step S5) is performed to sequentially remove the dissolved agent and the precipitating agent, thereby achieving the effective dissolution, precipitation, and lyophilization in the present embodiment. In this way, the APIs is allowable to be processed in the same reaction chamber during the above-mentioned steps, instead of being repeatedly transferred between various reaction chambers, or contacting with external environment. Thus, the manufacturing method of the present embodiment may effectively improve the possible contamination, dramatically reducing the residue, impurity or organic reagents remained in the medication, so that, the medication obtained thereby may meet the requirements of low toxicity and less contamination.

Also, further referring to the manufacturing method of the medication in the present embodiment, the lyophilization process is carried out on the crystals in the semi-solid dosage form, which is different from the general lyophilization process (including dissolution, sublimation, and crystallization), to precisely control the particle size of the medication. For example, when the API includes triamcinolone acetonide, a PSD of the crystals in the medication is controlled at about 40-50µm, with 90% of the crystals having a particle size (D90) of less than 45µm. On the other hand, when the API includes the YC-1 compound, a PSD of the crystals in the medication is controlled at about 60-80µm, with 10% of the crystals having a particle size (D10) of less than 1µm, with 50% of the crystals having a particle size (D50) of less than 20µm, and with 90% of the crystals having a particle size (D90) of less than 70µm, to meet the GMP standard for medication.

Please refer to FIG. 3 to FIG. 9, which are several examples of the manufacturing method of the medication in the present disclosure, with the related data being provided in the following paragraphs for verifying the functions and the advantages thereof.

### Example 1

In the example 1, 40 mg of triamcinolone acetonide is dissolved in 2.5 ml of methanol (if necessary, the methanol may be heated to 32°C for fast dissolution) in a round-bottom flask to obtain a solution, and the solution is filtered through a filter membrane to collect a filtrate into a precipitation bottle. Next, 4 ml of filtered pure water is added into the precipitation bottle at the room temperature, and the filtrate within the precipitation bottle is continuously stirred for 1 hour for completely precipitating crystals. Then, the first concentration step S4 (stirring for 20-24 hours to remove the methanol) and the second concentration step S5 (stirring for 12 to 16 hours to remove the pure water) are performed while continuously stirring the filtrate within the precipitation bottle, followed by quantifying a volume of the crystal and primary packaging the crystal while continuously stirring the filtrate within the precipitation bottle. After that, the vacuum concentration process is performed for another 12 to 16 hours to further remove the pure water. Then, the PSD of the crystals in a medication obtained thereby is about 10-100µm, with 10% of the crystals having a particle size (D10) of less than 25.839µm, with 50% of the crystals having a particle size (D50) of less than 50.817µm, and with 90% of the crystals having a particle size (D90) of less than 91.933µm, as shown in FIG. 3.

### Example 2

In the example 2, 40 mg of triamcinolone acetonide is dissolved in 2.5 ml of ethanol (if necessary, the ethanol may be heated to 32°C for fast dissolution) in a round-bottom flask to obtain a solution, and the solution is filtered through a filter membrane to collect a filtrate into a precipitation bottle. Next, 4 ml of filtered pure water is added into the precipitation bottle at the room temperature, and the filtrate within the precipitation bottle is continuously stirred for 1 hour for completely precipitating the crystals. Then, the first concentration step S4 (stirring for 20-24 hours to remove the ethanol) and the second concentration step S5 (stirring for 12 to 16 hours to remove the pure water) are performed while continuously stirring the filtrate within the precipitation bottle, followed by quantifying a volume of the crystal and primary packaging the crystal while continuously stirring the filtrate within the precipitation bottle. After that, the vacuum concentration process is performed for another 12 to 16 hours to further remove the pure water. Then, the PSD of the crystals in a medication obtained thereby is about 1-100µm, with 10% of the crystals having a particle size (D10) of less than 4.723µm, with 50% of the crystals having a particle size (D50) of less than 12.828µm, and with 90% of the crystals having a particle size (D90) of less than 31.582µm, as shown in FIG. 4.

### Example 3

In the example 3, 40 mg of triamcinolone acetonide is dissolved in 2.5 ml of acetone (if necessary, the acetone may be heated to 32°C for fast dissolution) in a round-bottom flask to obtain a solution, and the solution is filtered through a filter membrane to collect a filtrate into a precipitation bottle. Next, 4 ml of filtered pure water is added into the precipitation bottle at the room temperature, and the filtrate within the precipitation bottle is continuously stirred for 1 hour for completely precipitating the crystals. Then, the first concentration step S4 (stirring for 20-24 hours to remove the acetone) and the second concentration step S5 (stirring for 12 to 16 hours to remove the pure water) are performed while continuously stirring the filtrate within the precipitation bottle, followed by quantifying a volume of the crystal and primary packaging the crystal while continuously stirring the filtrate within the precipitation bottle. After that, the vacuum concentration process is performed for another 12 to 16 hours to further remove the pure water. Then, the PSD of the crystals in a medication obtained thereby is about 10-100µm, with 10% of the crystals having a particle size (D10) of less than 34.313 µm, with 50% of the crystals having a particle size (D50) of less than 64.922µm, and with 90% of the crystals having a particle size (D90) of less than 115.052µm, as shown in FIG. 5.

### Example 4

In the example 4, 1200 mg of triamcinolone acetonide is dissolved in 75 ml of ethanol (if necessary, the ethanol may be heated to 32°C for fast dissolution) in a round-bottom flask to obtain a solution, and the solution is filtered through a filter membrane to collect a filtrate into a precipitation bottle. Next, 120 ml of filtered pure water is added into the precipitation bottle at the room temperature, and the filtrate within the precipitation bottle is continuously stirred for 1 hour for completely precipitating the crystals. Then, the first concentration step S4 (stirring for 20-24 hours to remove the ethanol) and the second concentration step S5 (stirring for 12 to 16 hours to remove the pure water) are performed while continuously stirring the filtrate within the precipitation bottle, followed by quantifying a volume of the crystal and primary packaging the crystal while continuously stirring the filtrate within the precipitation bottle. After that, the lyophilization process is performed under a pressure of about 0.5 mmHg and a temperature gradually increasing from -35 degrees Celsius to zero degrees Celsius and then to 35 degrees Celsius (-35°C→0°C→35°C) for 22 to 24 hours to further remove the pure water. Then, the PSD of the crystals in a medication obtained thereby is about 1-100µm, with 10% of the crystals having a particle size (D10) of less than 5.617µm, with 50% of the crystals having a particle size (D50) of less than 15.848µm, and with 90% of the crystals having a particle size (D90) of less than 35.262µm, as shown in FIG. 6.

### Example 5

In the example 5, 40 mg of triamcinolone acetonide is dissolved in 2.5 ml of tert-butanole (if necessary, the tert-butanole may be heated to 30°C for fast dissolution) in a round-bottom flask to obtain a solution, and the solution is filtered through a filter membrane to collect a filtrate into a precipitation bottle. Next, 8 ml of filtered pure water is added into the precipitation bottle at the room temperature, and the filtrate within the precipitation bottle is continuously stirred for 1 hour for completely precipitating the crystals. Then, the first concentration step S4 (stirring for 20 to 24 hours to remove the tert-butanole) and the second concentration step S5 (stirring for 12 to 16 hours to remove the pure water) are performed while continuously stirring the filtrate within the precipitation bottle, followed by quantifying a volume of the crystal and primary packaging the crystal while continuously stirring the filtrate within the precipitation bottle. After that, the vacuum concentration process is performed for another 12 to 16 hours to further remove the pure water. Then, the PSD of the crystals in a medication obtained thereby is about 1-100µm, with 10% of the crystals having a particle size (D10) of less than 5.488µm, with 50% of the crystals having a particle size (D50) of less than 15.824µm, and with 90% of the crystals having a particle size (D90) of less than 36.362µm, as shown in FIG. 7.

### Example 6

In the example 6, 40 mg of triamcinolone acetonide is dissolved in 4 ml of isopropanol (if necessary, the isopropanol may be heated to 30°C for fast dissolution) in a round-bottom flask to obtain a solution, and the solution is filtered through a filter membrane to collect a filtrate into a precipitation bottle. Next, 9 ml of filtered pure water is added into the precipitation bottle at the room temperature, and the filtrate within the precipitation bottle is continuously stirred for 1 hour for completely precipitating the crystals. Then, the first concentration step S4 (stirring for 20-24 hours to remove the isopropanol) and the second concentration step S5 (stirring for 12 to 16 hours to remove the pure water) are performed while continuously stirring the filtrate within the precipitation bottle, followed by quantifying a volume of the crystal and primary packaging the crystal while continuously stirring the filtrate within the precipitation bottle. After that, the vacuum concentration process is performed for another 12 to 16 hours to further remove the pure water. Then, the PSD of the crystals in a medication obtained thereby is about 10-100µm, with 10% of the crystals having a particle size (D10) of less than 15.709µm, with 50% of the crystals having a particle size (D50) of less than 29.590µm, and with 90% of the crystals having a particle size (D90) of less than 53.806µm, as shown in FIG. 8.

### Example 7

In the example 7, 1800 mg of the YC compound is dissolved in 40 ml of ethanol (if necessary, the ethanol may be heated to 32°C for fast dissolution) in a round-bottom flask to obtain a solution, and the solution is filtered through a filter membrane to collect a filtrate into a precipitation bottle. Next, 50 ml of filtered pure water is added into the precipitation bottle at the room temperature, and the filtrate within the precipitation bottle is continuously stirred for 1 hour for completely precipitating the crystals. Then, the first concentration step S4 (stirring for 20 to 24 hours to remove the ethanol) and the second concentration step S5 (stirring for 12 to 16 hours to remove the pure water) are performed while continuously stirring the filtrate within the precipitation bottle, followed by quantifying a volume of the crystal and primary packaging the crystal while continuously stirring the filtrate within the precipitation bottle. After that, the vacuum concentration process is performed for another 12 to 16 hours to further remove the pure water. Then, the PSD of the crystals in a medication obtained thereby is about 0.1-110µm, with 10% of the crystals having a particle size (D10) of less than 0.188µm, with 50% of the crystals having a particle size (D50) of less than 7.627µm, and with 90% of the crystals having a particle size (D90) of less than 54.780µm, as shown in FIG. 9.

### Example 8

In the example 8, 400 mg of the YC compound is dissolved in 35 ml of tert-butanole (if necessary, the tert-butanole may be heated to 30°C for fast dissolution) in a round-bottom flask to obtain a solution, and the solution is filtered through a filter membrane to collect a filtrate into a precipitation bottle. Next, 90 ml of filtered pure water is added into the precipitation bottle at the room temperature, and the filtrate within the precipitation bottle is continuously stirred for 1 hour for completely precipitating the crystals. Then, the first concentration step S4 (stirring for 20 to 24 hours to remove the tert-butanole) and the second concentration step S5 (stirring for 12 to 16 hours to remove the pure water) are performed while continuously stirring the filtrate within the precipitation bottle, followed by quantifying a volume of the crystal and primary packaging the crystal while continuously stirring the filtrate within the precipitation bottle. After that, the vacuum concentration process is performed for another 12 to 16 hours to further remove the pure water. Then, the PSD of the crystals in a medication obtained thereby is about 0.1-1000µm, with 10% of the crystals having a particle size (D10) of less than 0.294µm, with 50% of the crystals having a particle size (D50) of less than 11.950µm, and with 90% of the crystals having a particle size (D90) of less than 188.035µm, as shown in FIG. 10.

### Example 9

In the example 9, 400 mg of the YC compound is dissolved in 30 ml of isopropanol (if necessary, the isopropanol may be heated to 30°C for fast dissolution) in a round-bottom flask to obtain a solution, and the solution is filtered through a filter membrane to collect a filtrate into a precipitation bottle. Next, 65 ml of filtered pure water is added into the precipitation bottle at the room temperature, and the filtrate within the precipitation bottle is continuously stirred for 1 hour for completely precipitating the crystals. Then, the first concentration step S4 (stirring for 20 to 24 hours to remove the isopropanol) and the second concentration step S5 (stirring for 12 to 16 hours to remove the pure water) are performed while continuously stirring the filtrate within the precipitation bottle, followed by quantifying a volume of the crystal and primary packaging the crystal while continuously stirring the filtrate within the precipitation bottle. After that, the vacuum concentration process is performed for another 12 to 16 hours to further remove the pure water. Then, the PSD of the crystals in a medication obtained thereby is about 0.1-50µm, with 10% of the crystals having a particle size (D10) of less than 0.192µm, with 50% of the crystals having a particle size (D50) of less than 2.180µm, and with 90% of the crystals having a particle size (D90) of less than 10.803µm, as shown in FIG. 11.

### Example 10

In the example 10, 1600 mg of the YC compound is dissolved in 80 ml of ethanol (if necessary, the ethanol may be heated to 32°C for fast dissolution) in a round-bottom flask to obtain a solution, and the solution is filtered through a filter membrane to collect a filtrate into a precipitation bottle. Next, 100 ml of filtered pure water is added into the precipitation bottle at the room temperature, and the filtrate within the precipitation bottle is continuously stirred for 1 hour for completely precipitating the crystals. Then, the first concentration step S4 (stirring for 20 to 24 hours to remove the ethanol) and the second concentration step S5 (stirring for 12 to 16 hours to remove the pure water) are performed while continuously stirring the filtrate within the precipitation bottle, followed by quantifying a volume of the crystal and primary packaging the crystal while continuously stirring the filtrate within the precipitation bottle. After that, the lyophilization process is performed under a pressure of about 0.5 mmHg and a temperature gradually increasing from -20 degrees Celsius to zero degrees Celsius and then to 35 degrees Celsius (-20°C→0°C→35°C) for 22 to 24 hours to further remove the pure water. Then, the PSD of the crystals in a medication obtained thereby is about 0.5-100µm, with 10% of the crystals having a particle size (D10) of less than 0.717µm, with 50% of the crystals having a particle size (D50) of less than 15.572µm, and with 90% of the crystals having a particle size (D90) of less than 66.663µm, as shown in FIG. 12.

### Example 11

In the example 11, 800 mg of the YC compound is dissolved in 20 ml of acetone (if necessary, the acetone may be heated to 30°C for fast dissolution) in a round-bottom flask to obtain a solution, and the solution is filtered through a filter membrane to collect a filtrate into a precipitation bottle. Next, 30 ml of filtered pure water is added into the precipitation bottle at the room temperature, and the filtrate within the precipitation bottle is continuously stirred for 1 hour for completely precipitating the crystals. Then, the first concentration step S4 (stirring for 20 to 24 hours to remove the acetone) and the second concentration step S5 (stirring for 12 to 16 hours to remove the pure water) are performed while continuously stirring the filtrate within the precipitation bottle, followed by quantifying a volume of the crystal and primary packaging the crystal while continuously stirring the filtrate within the precipitation bottle. After that, the vacuum concentration process is performed for another 12 to 16 hours to further remove the pure water. Then, the PSD of the crystals in a medication obtained thereby is about 0.1-120µm, with 10% of the crystals having a particle size (D10) of less than 0.548µm, with 50% of the crystals having a particle size (D50) of less than 10.168µm, and with 90% of the crystals having a particle size (D90) of less than 120.467µm, as shown in FIG. 13.

### Example 12

In the example 12, 600 mg of the YC compound is dissolved in 20 ml of ethanol (if necessary, the ethanol may be heated to 32°C for fast dissolution) in a round-bottom flask to obtain a solution, and the solution is filtered through a filter membrane to collect a filtrate into a precipitation bottle. Next, 120 ml of filtered pure water is added into the precipitation bottle at the room temperature, and the filtrate within the precipitation bottle is continuously stirred for 1 hour for completely precipitating the crystals. Then, the first concentration step S4 (stirring for 20 to 24 hours to remove the ethanol) and the second concentration step S5 (stirring for 12 to 16 hours to remove the pure water) are performed while continuously stirring the filtrate within the precipitation bottle, followed by quantifying a volume of the crystal and primary packaging the crystal while continuously stirring the filtrate within the precipitation bottle. After that, the lyophilization process is performed under a pressure of about 0.5 mmHg and a temperature gradually increasing from -20 degrees Celsius to zero degrees Celsius and then to 35 degrees Celsius (-20°C→0°C→35°C) for 22-24 hours to further remove the pure water. Then, the PSD of the crystals in a medication obtained thereby is about 0.1-100µm, with 10% of the crystals having a particle size (D10) of less than 0.193µm, with 50% of the crystals having a particle size (D50) of less than 9.802µm, and with 90% of the crystals having a particle size (D90) of less than 58.439µm, as shown in FIG. 14.

In addition, please refer to the following Table 2 to Table 6, in which the weights of the medication obtained from the manufacturing methods of the first embodiment and the second embodiment are compared in further. Firstly, as shown in the Table 2, an effective dosage of the API (such as including triamcinolone acetonide) is processed through a dry powder filling technology in the manufacturing method of the first embodiment, with the API being filled with the sterilized dry powder, followed by quantify a volume or a weight thereof for primary packaging, thereby obtaining the medication according to the manufacturing method of the first embodiment. With these arrangements, five groups of the medications (including the numerals 1-1 to 1-5 as shown in Table 3) are obtain accordingly, and the weights (in grams, g) of the five groups of the medications in various processed steps of the manufacturing method of the medication are measured and listed in the following Table 3, for obtaining a coefficient variation (namely, a standard value/average value, CV) of the weights or the volumes between the medications within the five groups of the medications being about 3.57%.

Please further refer to the Table 2, the API (such as including triamcinolone acetonide) is firstly dissolved in the dissolved agent (the preparation step S 1), followed by sequentially performing the sterilization (the sterilization step S2) and the precipitation (the precipitation step S3) processes, to obtain the crystals, and then, the multi-stepped vacuum concentration process (including the first concentration step S4 and the second concentration step S5) is performed to sequentially remove the dissolved agent and the precipitating agent, thereby achieving the effective dissolution, precipitation, and lyophilization in the manufacturing method of the second embodiment. Then, the primary packaging step S6 and the drying step S7 are sequentially performed, by firstly quantifying a volume of the crystals to primary packaging and refilling the crystals, and next removing the residual precipitating agent through the lyophilization process and the vacuum concentration process, thereby obtaining the medication according to the manufacturing method of the second embodiment. With these arrangements, several groups of the medications (including the numeral 2-1 to 2-10 as shown in Table 4, the numerals 3-1 to 3-19 as shown in Table 5, and the numerals 4-1 to 4-12 as shown in Table 6) are obtain accordingly, and the weights (in grams) of these groups of the medications in various processed steps of the manufacturing method of the medication are measured and listed in the following Table 4, the Table 5 and the Table 6, for obtaining a coefficient variation (namely, a standard value/average value, CV) of the weights or the volumes between the medications within these groups of the medications being about 0.29-1.12%.

**Table 2: Comparison of the manufacturing methods of the medicament in the first embodiment and in the second embodiment of the present disclosure**

| The manufacturing method in the first embodiment | | The manufacturing method in the second embodiment | |
|---|---|---|---|
| steps | results | steps | results |
| sampling | with the APIs probably mixing with tiny hairs, impurities, or other contaminations | sampling | with the APIs probably mixing with tiny hairs, impurities, or other contaminations |
| none | - | preparation step S1 | with the APIs being dissolved; and with the APIs being sterilized and filtered, by removing the contaminations like hairs, impurities, residues, or microorganisms therefrom |
| none | - | precipitation step S3 | with the APIS being recrystallized and purified, and with the particle size of the crystals being uniform and microminiaturized |
| none | - | first concentration step S4 and second concentration step S5 | removing most of the dissolved agent and the precipitating agent |
| Primary packaging | With relative higher CV and relative lower efficiency | primary packaging step S6 | with uniform particle size being easy to refill |
| none | - | drying step S7 | completely removing the dissolved agent and the precipitating agent, and drying the crystals with stable quality |

In other words, the medications according to the manufacturing method of the second embodiment is allowable to be dried either when the crystals is incomplete drying or complete drying, and the micro-volume or the weight of the crystals can be precisely quantified for primary packaging and filling, wherein the CV (for example being about 0.29-1.12%) of the weights or the volumes between the medications obtained in the second embodiment is dramatically lower than the CV (for example being about 3.57%) of the weights or the volumes between the medications obtained in the first embodiment. Thus, the medications of the second embodiment enable to meets the requirement of finer particle, so as to achieve better product quality.

**Table 3: Weights (in grams) of medications obtained in the manufacturing method of medication according to the first embodiment**

| numbers | Bottle, capsule, and traditional powder | Bottle and capsule | Traditional powder |
|---|---|---|---|
| 1-1 | 12.7559 | 12.7148 | 0.0411 |
| 1-2 | 12.6612 | 12.6196 | 0.0416 |
| 1-3 | 12.5409 | 12.5002 | 0.0407 |
| 1-4 | 12.7548 | 12.7166 | 0.0382 |
| 1-5 | 12.6459 | 12.6041 | 0.0418 |
| standard value | 0.001451551 | | |
| average value | 0.04068 | | |
| CV | 3.57% | | |

**Table 4: Weights (in grams) of medications obtained in the manufacturing method of medication according to the second embodiment**

| numbers | Bottle, and capsule | Bottle, capsule, and lyophilization | lyophilization |
|---|---|---|---|
| 2-1 | 12.8484 | 12.8884 | 0.0400 |
| 2-2 | 12.8844 | 12.9242 | 0.0398 |
| 2-3 | 13.0044 | 13.0444 | 0.0400 |
| 2-4 | 13.2111 | 13.2520 | 0.0409 |
| 2-5 | 13.1828 | 13.2245 | 0.0417 |
| 2-6 | 13.3191 | 13.3599 | 0.0408 |
| 2-7 | 13.2211 | 13.2623 | 0.0412 |
| 2-8 | 13.1945 | 13.2362 | 0.0417 |
| 2-9 | 13.1521 | 13.1934 | 0.0413 |
| 2-10 | 13.1765 | 13.2174 | 0.0409 |
| standard value | 0.00011547 | | |
| average value | 0.0399 | | |
| CV | 0.29% | | |

**Table 5: Weights (in grams) of medications obtained in the manufacturing method of medication according to the second embodiment**

| numbers | Bottle, and capsule | Bottle, capsule, and lyophilization | lyophilization |
|---|---|---|---|
| 3-1 | 13.2851 | 13.3248 | 0.0397 |
| 3-2 | 13.3321 | 13.3728 | 0.0407 |
| 3-3 | 13.288 | 13.3280 | 0.0400 |
| 3-4 | 13.4261 | 13.4667 | 0.0406 |
| 3-5 | 13.2474 | 13.2881 | 0.0407 |
| 3-6 | 13.2733 | 13.3137 | 0.0404 |
| 3-7 | 13.3709 | 13.4112 | 0.0403 |
| 3-8 | 13.2911 | 13.3308 | 0.0397 |
| 3-9 | 13.3201 | 13.3606 | 0.0405 |
| 3-10 | 13.3999 | 13.4406 | 0.0407 |
| 3-11 | 13.2605 | 13.3008 | 0.0403 |
| 3-12 | 13.3343 | 13.3744 | 0.0401 |
| 3-13 | 13.3533 | 13.3933 | 0.0400 |
| 3-14 | 13.3206 | 13.3606 | 0.0400 |
| 3-15 | 13.2321 | 13.272 | 0.0399 |
| 3-16 | 13.4771 | 13.5163 | 0.0392 |
| 3-17 | 13.4476 | 13.4870 | 0.0394 |
| 3-18 | 13.3156 | 13.3553 | 0.0397 |
| 3-19 | 13.4272 | 13.4677 | 0.0405 |
| standard value | 0.00045073 | | |
| average value | 0.0401 | | |
| CV | 1.12% | | |

**Table 6: Weights (in grams) of medications obtained in the manufacturing method of medication according to the second embodiment**

| numbers | Bottle, and capsule | Bottle, capsule, and lyophilization | lyophilization |
|---|---|---|---|
| 4-1 | 13.2816 | 13.3209 | 0.0393 |
| 4-2 | 12.9948 | 13.0338 | 0.0390 |
| 4-3 | 13.2458 | 13.2855 | 0.0397 |
| 4-4 | 13.2256 | 13.2648 | 0.0392 |
| 4-5 | 13.2278 | 13.2674 | 0.0396 |
| 4-6 | 13.1487 | 13.1884 | 0.0397 |
| 4-7 | 13.1834 | 13.2227 | 0.0393 |
| 4-8 | 13.3649 | 13.4046 | 0.0397 |
| 4-9 | 13.1149 | 13.1548 | 0.0399 |
| 4-10 | 13.0727 | 13.1126 | 0.0399 |
| 4-11 | 13.0766 | 13.1164 | 0.0398 |
| 4-12 | 13.2134 | 13.2529 | 0.0395 |
| standard value | 0.000290767 | | |
| average value | 0.0396 | | |
| CV | 0.74% | | |

Overall speaking, according to the manufacturing method of a medication of the present disclosure, a special crystal precipitation technique and a lyophilization process are combined in use to precisely define the particle size of precipitated crystals, in which, the multi-solvents are applied to the active pharmaceutical ingredients for crystal precipitation, and the lyophilization process is carried out on a semi-solid dosage form, thereby simplifying the manufacturing process of the medication, and effectively preventing from any external contamination at the same time. Thus, the manufacturing method of the present disclosure enables to process water indissoluble APIs such as triamcinolone acetonide or the YC-1 compound or the antibiotic drugs, to manufacture into the injections which can be absorbed and utilized by human or animals, in particular the injections with higher purity, less contamination and lower toxicity, for example an ophthalmic injection for a treatment of vitreous visualization in ophthalmic vitrectomy, or diabetic macular edema. Through these arrangements, the medication obtained from the manufacturing method of a medication of the present disclosure is allowable to gain better product quality.

## Claims

1. A manufacturing method of a medication, **characterized by** comprising:
dissolving an effective dosage of an active pharmaceutical ingredient in a dissolved agent to form a first solution, wherein the active pharmaceutical ingredient comprises triamcinolone acetonide or YC-1 compound;
filtering the first solution through a filter membrane at a room temperature to obtain a second solution;
adding a precipitating agent into the second solution to obtain a third solution, and continuously stirring the third solution to precipitate crystals;
further stirring the third solution under the room temperature and a low pressure to sequentially remove the dissolved agent and the precipitating agent from the third solution;
quantifying a volume of the crystals to primary package and to refill the crystals; and
performing a lyophilization process on the crystals to obtain a medication, wherein the triamcinolone acetonide is represented by structure of formula (I): 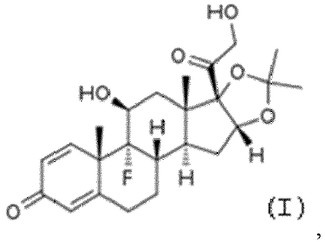
and the YC-1 compound is represented by structure of formula (II):

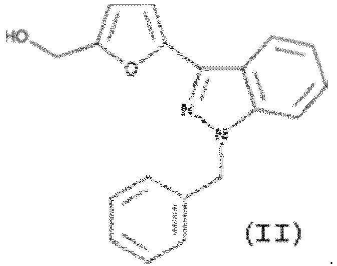

2. The manufacturing method of the medication according to claim 1, **characterized in that** the room temperature is 20 to 30 degrees Celsius.

3. The manufacturing method of the medication according to any one of claims 1-2, **characterized in that** an aperture of the filter membrane is 0.22 to 0.45 micrometers.

4. The manufacturing method of the medication according to any one of claims 1-3, **characterized in that** the dissolved agent is selected from a group consisting of (C1-C4) alcohol and (C1-C4) ketone.

5. The manufacturing method of the medication according to any one of claims 1-4, **characterized in that** the dissolved agent is selected from a group consisting of methanol, ethanol, isopropanol, tertiary butanol and acetone.

6. The manufacturing method of the medication according to any one claims 1-5, **characterized in that** the precipitating agent comprises pure water.

7. The manufacturing method of the medication according to any one of claims 1-6, **characterized in that** a volume ratio of the precipitating agent to the second solution is 2:1.

8. The manufacturing method of the medication according to any one of claims 1-7, **characterized in that** a ratio of a weight of the active pharmaceutical ingredient to a volume of the dissolved agent is 10 mg: 0.5 to 0.8 ml.

9. The manufacturing method of the medication according to any one of claims 1-8, **characterized in that** a ratio of a weight of the active pharmaceutical ingredient to a volume of the dissolved agent is 10 mg: 0.65 ml.

10. The manufacturing method of the medication according to any one of claims 1-9, **characterized in that** a ratio of a weight of the active pharmaceutical ingredient to a volume of the precipitating agent is 10 mg: 0.5 to 1.5 ml.

11. The manufacturing method of the medication according to any one of claims 1-10, **characterized in that** a ratio of a weight of the active pharmaceutical ingredient to a volume of the precipitating agent is 10 mg: 1 ml.

12. The manufacturing method of the medication according to any one of claims 1-11, **characterized by**:
performing a first concentration step by stirring the third solution at the room temperature and under a pressure of -720 to -685mmHg, to remove the dissolved agent; and
performing a second concentration step by stirring the third solution at the room temperature and under a pressure of -730 to -710mmHg, to remove the precipitating agent.

13. The manufacturing method of the medication according to claim 12, **characterized in that** an operating time of the first concentration step is 22 to 24 hours, and an operating time of the second concentration step is 12 to 16 hours.

14. The manufacturing method of the medication according to any one of claims 1-13, **characterized in that** the active pharmaceutical ingredient comprises triamcinolone acetonide, and 90% of the crystals has a particle size distribution (D90) of 40 to 50 micrometers.

15. The manufacturing method of the medication according to any one of claims 1-13, **characterized in that** the active pharmaceutical ingredient comprises YC-1 compound, and 90% of the crystals have a particle size distribution (D90) of 60 to 80 micrometers.
